# EUROPEAN PATENT APPLICATION

(11) **EP 1 627 641 A1**
(43) Date of publication of application: **22.02.2006**
(21) Application number: 04018489.7
(22) Date of filing: 04.08.2004
(51) Int. Cl.: A61K 33/26, A61P 31/00

(54) **Pharmaceutical compositions comprising metal oxides and uses thereof**

(71) Applicant: Eluwa, Victor, 81675 München (DE)
(72) Inventor: Eluwa, Victor, 81675 München (DE)

(57) **Abstract**

Pharmaceutical compositions comprising at least one metal oxide such as Fe₂O₃ are provided. Furthermore, therapeutic regimen for using such compositions for the prevention, treatment, and/or amelioration of conditions, in particular of viral diseases and symptoms thereof, especially AIDS (Acquired Immune Deficiency Syndrome) and AIDS-related diseases are described.

## Description

### Field of the invention

The present invention relates to pharmaceutical compositions including at least one metal oxide and therapeutic regimen for using such compositions for the prevention, treatment, and management of conditions, in particular of viral diseases and symptoms thereof, especially AIDS (Acquired Immune Deficiency Syndrome) and AIDS-related diseases.

### Background of the invention

Since 1983, it has been explicated that HIV forms the cause of AIDS (Acquired Immune Deficiency Syndrome) and AIDS-related diseases. At the beginning of its discovery, HIV had been differently named LAV (Lymphoadenopathy Associated Virus) by French researchers, or HTLV-III (Human T-lymphotropic Virus Type III) or ARV (AIDS-Related Virus) by American researchers. However, it was determined in 1986 that the designation of the virus is unified into HIV. WHO predicts that AIDS patients and HIV-infected persons in the world have reached ten millions and forty millions, respectively, by 2000.

As described above, AIDS now grows into a great foe of the human. However, no basic therapy has been found yet. Azidothymidine (AZT; 3'-azido-3'-deoxythymidine) is one of the few drugs that have been approved up to the present (Mitsuya, et al., Proc. Natl. Acad. Sci. USA 82 (1985), 7096-7100). AZT has been confirmed in its effectiveness by what life-prolonging effect is recognized to a significant extent, and so on. On the other hand, it has been said that a myelodepressant effect is remarkably observed as a side effect in addition to headache and gastrointestinal disorders, and long-term administration hence offers a problem (Richmann et al., New Engl. J. Med. 317 (1987), 185-197). AZT has also been said that it is of no effect on cells already infected with HIV, and although it can prevent the crisis of AIDS-related complex, it is directly useless for remedy after infection.

In addition to AZT, many anti-HIV agents including nucleic acid derivatives, soluble CD4 and the like have been researched and developed. As literature related to the present invention, there are documents which describe porphyrin or chemically modified plasma proteins as well as integrase and protease inhibitors, respectively, as having an anti-HIV activity.

However, so far most, if not all, of the currently investigated anti-HIV drugs suffer from the drawback of having severe side effects. Furthermore, the anti-HIV activities of the drugs are often only against the infection and proliferation of HIV, but have no killing effect on cells persistently infected with HIV.

Thus, there is a need for pharmaceutical compositions and therapeutic regimen for the treatment of HIV and conditions arising from HIV infection. In accordance with this, it is desirable that the pharmaceutical composition is also effective against opportunistic infections and diseases such as infection by cytomegalovirus, hepatitis virus C, and herpes zoster virus; fungal infections such as candidias; bacterial infections; tumors such as non-Hodgkin's lymphoma; as well as other diseases including disorders of the immune system.

The solution to said technical problem is achieved by providing the embodiments characterized in the claims, and described further below.

### Description of the invention

The present invention relates to the use of metal oxides, in particular of metal(III) oxides for the treatment of diseases and conditions thereof, in particular of viral diseases such as HIV infection. More particularly, the present invention relates to pharmaceutical compositions suitable for the treatment of HIV/AIDS as well as of diseases that are also commonly associated with AIDS such as infections by other pathogens and cancer.

The present invention is based on the observation that patients suffering from AIDS already in a late stage could be cured with the use of moderate amounts of a metal oxide, i.e. iron(III) oxide (Fe₂O₃). As described in the example, it could surprisingly be found that after therapy of approximately 6 to 8 weeks all patients were not only symptom-free, but also appeared to be free of the HIV virus as well as of other viruses such as hepatitis virus C and cytomegalovirus. Even anti-HIV antibodies could no longer be detected. Hence, using conventional HIV tests, those patients would be diagnosed as HIV negative.

Furthermore, after the treatment period the amount of candida albicans female patients could be reduced to a normal level, and other opportunistic infections as well as other conditions such as metastatic growth and alteration of the skin associated, for example, with Kaposi's sarcoma could be ameliorated.

Thus, in accordance with the present invention it is envisaged that pharmaceutical compositions comprising metal oxides such as iron(III) oxide are not only suitable for the treatment of viral diseases, in particular AIDS, but may also be generally be used as anti-pathogenic agents such as anti-microbial, anti-bacterial, anti-algal agents, or a combination thereof. Likewise, it is envisaged that the compositions described herein may be used for preventing, treating, and/or managing various conditions that are non-pathogenic. For example, non-pathogenic conditions are believed to include certain autoimmune disorders, neurological disorders, and circulatory disorders. While the exact mechanism of the activity of such compounds and compositions is not described herein, nonetheless suitable prevention, treatment, and/or management of such conditions may be obtained by administering the compounds or compositions of the invention as will be readily apparent to one of ordinary skill in the art.

Without intending to be bound by theory it is assumed that the metal oxides referred to herein, in particular the metal oxides in a common oxidative state such as preferably me⁺³ and more specifically Fe₂O₃ do not immediately influence viruses or other agents, but rather confer resistance to the cells and a positive effect on metabolic processes, quasi harmonizing them, either as catalyst for other compounds or independently. Furthermore, in therapy, it is recommend an at least largely vegetarian diet, as the abundantly contained secondary substances are expected to develop therapy-enhancing synergies with the metal oxide composition.

Previously, international application WO95/35113 claimed the use of iron(III) oxide and/or its hydroxide for the treatment of AIDS, wherein a dosage unit of 400 mg iron(III) oxide with a particle size of 5 µm is suggested for the daily therapy by oral administration. However, the claimed use of iron(III) oxide was based on in vitro experiments only and without a suitable control which could distinguish between a specific pathogen-inhibitory effect and a general unspecific cellular response upon the exposure to a metal, which actually reflects the toxicity of the metal oxide when applied in vivo. Furthermore, it was generally held in the prior art that metal(III) oxides, in particular iron(III) oxide (Fe₂O₃), i.e. ferric hydroxide and ferric oxide, are not absorbed by the human body. Accordingly, the teaching in WO95/35113 is of speculative nature and apparently has never been tried to put into practice.

As described in the Example experiments performed in accordance with the present invention and which have been made in order to evaluate the reliability of the disclosure of this international application revealed that, as expected, the daily administration of 400 mg iron(III) oxide resulted in severe side effects making it impossible to further pursue the clinical study.

Thus, also experimental examination of the teaching of international application WO95/35113 would have led the person skilled in the art to conclude that this teaching was in error or an artefact. In any case, the use of 400 mg iron(III) oxide turned out to be substantially disadvantageous such that a corresponding dosage regimen could not be pursued as long as it would have been necessary in order to successfully treat the AIDS Disease, i.e. approximately at least four weeks or more. Accordingly, in one embodiment, the use of iron(III) oxide in a dosage unit of 400 mg as specifically taught in international application WO95/35113 is disclaimed herewith.

Furthermore, international application WO01/49303 claimed the use of compounds that have at least two polyvalent cations, at least one of which has a first valence state and at least one of which has a second, different valence state, such as Fe(II, III) oxide, in methods for prevention, management or treatment of a disease or condition thereof, wherein said compounds should be in a certain crystalline state. Though this international application provides a general description of different dosage and administration regimens, the examples are confined to in vitro experiments concerning the anti-pathogenic efficacy of the compounds on different kinds of bacteria. However, whether the claimed compounds are effective in vivo and, for example, on viruses as well, is not disclosed. Furthermore, no specific disclosure on the actual dosage to be used for the in vivo treatment of a patient suffering from a particular disease is given. Nevertheless, in one embodiment its disclosure, in particular the use of at least one compound or a pharmaceutically acceptable derivative thereof, that has at least two polyvalent cations, at least one of which as a first valence state and at least one of which has a second, different valence state, wherein said compound comprises a metal oxide, i.e. bismuth, cobalt, copper, iron, manganese, or praseodymium, are specifically disclaimed for the therapeutic applications disclosed in WO01/49303.

In this context, it should be emphasized that contrary to the two mentioned international applications and related applications, the present application provides evidence of the utility of the claimed metal oxide compounds and concomitantly also provides safe and applicable dosage and administration regimen, which lead to the claimed therapeutic effect.

Thus, the present invention provides a pharmaceutical composition preferably suitable for the treatment of a viral disease in a subject, said composition comprising in a dosage unit as the effective ingredient a metal oxide, and a pharmaceutically acceptable carrier. In one embodiment, the pharmaceutical composition of the present invention excludes a dosage unit which consists of 400 mg Fe₂O₃ with a particle size of about 5 micron when intended for use in the treatment of AIDS. Preferably, The pharmaceutical composition is substantially free of biologically active agents other than the metal oxides.

As used herein, the term "metal oxide" generally refers to any oxidized metal, wherein the term "metal" refers to any metal as commonly understood in accordance with the periodic table of the elements including the lanthanides and actinides, i.e. the elements with the atomic No. 58-71 and 90-103, respectively. While generally encompassed by the term "metal" alkali and alkaline earth metals are usually less preferred metals to be used in accordance with the present invention. Generally, any metal oxide may be suitable for use in accordance with present invention, for example aluminum oxide (Al₂O₃), antimony oxide (Sb₂O₃), bismuth oxide (Bi₂O₃), boron oxide (B₂O₃), indium oxide (In₂O₃), lanthanum oxide (La₂O₃), lutetium oxide (Lu₂O₃), scandium oxide (Sc₂O₃), titanium oxide/ dioxide (TiO & TiO₂), and other. Preferably, the metal elements are selected from the iron group, i.e. iron, cobalt and nickel, with iron being most preferred.

Furthermore, in one embodiment the term "metal oxide" also includes corresponding metal hydroxide compounds such as, for example, Fe(OH)₃. Essential for the oxidized metal compound to be used in the present invention is that the oxidation state, i.e. valence state of the metal is equivalent with that of the metal in the metal oxide described hereinbefore. Accordingly, even the use of metal compounds may be envisaged, wherein the "oxygen" component is fully or partly replaced by one or more different electronegative elements.
In addition, though less preferred and in one embodiment excluded for the scope of the present invention, the term "metal oxide" may include metal compounds which have at least two polyvalent cations, at least one of which has a first valence state and at least one of which has a second, different valence state, such as Fe(II, III) oxide (Fe₃O₄) as described for example in WO01/49303 and corresponding hydroxide compounds.

As demonstrated in the example, a metal oxide with a common valence state, i.e. iron(III) oxide, has been used and shown to be effective in the treatment of HIV/AIDS and conditions associated with AIDS. Accordingly, preferred metal oxides include those which have only one polyvalent cation with one particular valence state such as iron(III) and iron(III) oxide. In case the metal is referred to in the periodic table of elements with more than one valence state, i.e. ion charge, it is preferred in the pharmaceutical compositions of the present invention to use the metal compound in its highest naturally occurring valence state such as +3 for iron. While the exact mechanism of action of the oxidized metal used in accordance with the present invention may not be known, it is nevertheless assumed that metal with the valence state +3 such as iron(III) in Fe₂O₃ are particularly suitable for the purposes of the present invention. Therefore, metal(III) oxides are the most preferred compounds to be used in the pharmaceutical compositions described herein. One of ordinary skill in the art understands that, in general, the valence state of a species, such as a metal cation, is related to the charge associated with or assigned to the species.

Metal oxides are commercially available or can be produced by methods well known in the art. For example, pharmaceutically acceptable iron(III) oxide (Fe₂O₃) also known as ferric oxide, hematite, and red iron oxide is available as a natural or synthetic product from, e.g., Reade Advanced Materials, US, and Sigma-Aldrich. Similarly, other suitable metal oxides such as aluminum oxide, bismuth oxide, manganese oxide/dioxide, rare earth oxides, etc., see supra, are available from those companies as well as from others.

The term "AIDS" generally refers to a disease, at least moderately predictive of a defect in cell-mediated immunity, occurring in a person being infected with a virus that is causative for the diminished resistance to that disease. Such diseases include Kaposi's sarcoma, Pneumocystis carinii pneumonia and serious other opportunistic infections. These infections include pneumonia, meningitis or encephalitis due to various pathogen infections and/or cellular aberrations. As demonstrated in the example, the pharmaceutical compositions are particularly suited for the treatment of AIDS and any other immune deficiency caused by the human immunodeficiency virus (HIV). Accordingly, when stating that the pharmaceutical compositions of the present invention are used for the treatment of AIDS, it is preferably meant that HIV/AIDS is being treated, i.e. that the treatment results in a diminished level or preferably complete loss, i.e. below detectable levels, of the virus.

The pharmaceutical compositions of the present invention though preferably being suited for the treatment of a viral disease such as HIV/AIDS, are intended to be useful for preventing, treating, or managing any disease in a subject including, but not limited to viral infections, bacterial infections, cancer, autoimmune diseases, vascular diseases, disorder of metabolism, and the like, as well as any condition associated with such a disease. The at least one metal oxide compound or a pharmaceutically acceptable derivative thereof is preferably administered in an amount and for a period of time which is therapeutically effective to treat such a disease or condition (s).

The terms "patient" or "subject" as used herein refer to animals, particularly to mammals. In a preferred embodiment, the terms "patient" or "subject" refer to humans.

As used herein, the terms "adverse effects", "adverse side effects" and "side effects" include, but are not limited to, staining of the skin, headache, dry mouth, constipation, diarrhea, gastrointestinal disorders, dry skin, staining of the skin, hepatomegaly, fever, fatigue, and the like.

The phrase "therapeutically effective amount" when used herein in connection with the compositions and methods of the invention, means that amount of metal oxide composition, or a derivative thereof, which, alone or in combination with other drugs or treatment modalities, provides a therapeutic benefit in the prevention, treatment, or management, of one or more forms of HIV/AIDS or a symptom or related condition thereof. Preferably, the therapeutically effective amount of a component yields the desired therapeutic benefit without undue adverse side effects (such as toxicity, irritation or allergic response) commensurate with a reasonable benefit/risk ratio when used in the manner of this invention.

The term "substantially free" means less than about 10 weight percent, preferably less than about 5 weight percent, more preferably less than about 1 weight percent, and most preferably less than about 0.1 weight percent of therapeutically effective substances other than the metal oxides as defined herein and/or of added oxidative agents such as persulfate is present in the pharmaceutical composition according to the invention.

The term "controlled-release component" in the context of the present invention is defined herein as a compound or compounds, including polymers, polymer matrices, gels, permeable membranes, liposomes, microspheres or the like, or a combination thereof, that facilitates the controlled release of the active ingredient (e.g., ferric oxide) in the pharmaceutical composition.

The term "about", as used herein, should generally be understood to refer to both numbers in a range of numerals. Moreover, all numerical ranges herein should be understood to include each whole integer within the range.

In a particularly preferred embodiment of the present invention said metal in the pharmaceutical composition is iron. As mentioned before, said metal oxide is preferably iron oxide Fe₂O₃ or hydrated iron oxide Fe₂O₃ (H₂O).

The metal oxide compound, such as Fe₂O₃, may be black in color, such that care must be taken when formulating suitable topical pharmaceutical compositions according to the invention to inhibit or avoid blackening or staining of the skin. Without being bound by theory, it is believed that larger amounts of the metal oxide composition promote increased staining. Thus, in one embodiment, the pharmaceutical compositions preferably have an insufficient amount of metal oxide compound to cause visible skin staining.

The concentration of the metal oxide dispersed in the pharmaceutical composition, for example in a carrier, ranges from about 0.1 to 99 % by weight, preferably from about 1 to 75%, more preferably from about 2.5 to 50 % by weight and most preferably from about 5 to 25 % by weight.

Any suitable route of administration may be employed for providing the patient with an effective dosage of metal oxide, or a pharmaceutically acceptable derivative thereof. The most suitable route in any given case will depend on the nature and severity of the condition being prevented, treated, or managed. One preferred route is orally, preferably via the ingestion. In this embodiment, a preferred route of administration is by a gelatin capsule or a tablet. The magnitude of a prophylactic or therapeutic dose of metal oxide composition(s), or a derivative thereof, in the acute or chronic management of diseases and disorders described herein will vary with the severity of the condition to be prevented, treated, or managed and the route of administration. For example, oral, mucosal (including vaginal and rectal), parenteral (including subcutaneous, intramuscular, bolus injection, and intravenous, such as by infusion), sublingual, transdermal, nasal, buccal, and like may be employed. Dosage forms include tablets, troches, lozenges, dispersions, suspensions, suppositories, solutions, capsules, soft elastic gelatin capsules, patches, and the like. The dose, and perhaps the dose frequency, will also vary according to the age, body weight, and response of the individual patient. Suitable dosing regimens can be readily selected by those of ordinary skill in the art with due consideration of such factors.

As described in the example, the metal oxide could be formulated in stomach-soluble hard gelatin capsules and thus be orally administered to the patient. Thus, oral administration, preferably by capsule or tablet, is the preferred route of application of the metal oxide in accordance with the present invention. The compositions, when applied orally, can be applied about 1 to 10, preferably about 4 to 8 times per day until the condition is suitably cured or satisfactorily controlled.

In general, the total daily dosage for the conditions described herein can be from about 1 mg to 500 mg of the metal oxide or derivative thereof, while in another embodiment, the daily dosage can be from about 2 mg to 200 mg of the metal oxide composition. A unit dosage can include, for example, 10 mg, 20 mg, 30 mg, 40 mg, 50 mg, 60 mg, 70 mg, 80 mg, 90 mg, 100 mg, 120 mg, 150 mg or 200 mg of metal oxide composition. Preferably, the active ingredient is administered in single or divided doses from 1 to 10, preferably 3 to 9, most preferably 4 to 8 times a day; see also the example. The actual dosage unit and administration regimen may be decided on the basis of the overall physical condition of the subject to be treated; see also supra and infra. Furthermore, as explained in the example the successive administration of multiple doses and/or the controlled release of the metal oxide composition over time are preferred over one single dose administration. In a particular preferred embodiment of the present invention the total dose of metal oxide, in particular if it concerns iron(III) oxide or the hydrated derivative thereof is below 400 mg a day, preferably it is below 350 mg and most preferably it does exceed 300 mg a day.

In a preferred embodiment of the pharmaceutical composition of the present invention said dosage unit comprises about 1 to 350 mg of the metal oxide, preferably about 10 to 50 mg, most preferably about 20 mg. Typically, said metal oxide is present in an amount sufficient to administering to the subject from about 0.1 to about 5 mg/kg/day of said metal oxide, preferably wherein said pharmaceutical composition is designed for administration twice to five times a day. In a particularly preferred embodiment of the pharmaceutical composition said metal oxide is present in said dosage unit from about 0.15 to about 0.5 mg/kg.

As mentioned before, in a preferred embodiment the compositions are administered by an oral route of administration. The oral dosage forms may be conveniently presented in unit dosage forms and prepared by any methods available to those of ordinary skill in the art of pharmacy.

In managing the patient, the therapy may be initiated at a lower dose, e. g., from about 1 mg, and increased up to the recommended daily dose or higher depending on the patient's global response. It is further recommended that children, patients over 65 years, and those with impaired renal or hepatic function, initially receive low doses when administered systemically, and that they be titrated based on individual response(s) and blood level(s). It may be necessary to use dosages outside these ranges in some cases, as will be apparent to those of ordinary skill in the art.

Furthermore, it is noted that the clinician or treating physician will know how and when to interrupt, adjust, or terminate therapy in conjunction with individual patient response.

In practical use, the metal oxide, or a derivative thereof, can be combined as the active ingredient in intimate admixture with a pharmaceutical carrier according to conventional pharmaceutical compounding techniques. The carrier may take a wide variety of forms and may include a number of components depending on the form of preparation desired for administration. The compositions of the present invention include, but are not limited to, suspensions, solutions and elixirs; aerosols; or carriers, including, but not limited to, starches, sugars, microcrystalline cellulose, diluents, granulating agents, lubricants, binders, disintegrating agents, and the like. Particular examples of such compounds as well s methods for the formulation of the metal oxide are given in, e.g., the standard pharmacy book Remington: The Science and Practice of Pharmacy (20^{th} Edition, 2000) by Gennaro et al. (eds.) ISBN 0-683-306472; see also international application WO03/086319 the disclosure content of which is incorporated herewith by reference.

Pharmaceutical compositions of the present invention may be orally administered in discrete pharmaceutical unit dosage forms, such as capsules, cachets, soft elastic or hard gelatin capsules, tablets, or aerosols sprays, each containing a predetermined amount of the active ingredient, as a powder or granules, or as a solution or a suspension in an aqueous liquid, a non-aqueous liquid, an oil-in-water emulsion, or a water-in-oil liquid emulsion. Such compositions may be prepared by any of the methods of pharmacy, but all methods include the step of bringing into association the active ingredient with the pharmaceutically acceptable carrier which constitutes one or more necessary ingredients. In general, the compositions are prepared by uniformly and intimately admixing the active ingredient with liquid carriers or finely divided solid carriers or both, and then, if necessary, shaping the product into the desired presentation. Suitable types of oral administration include oral solid preparations, such as capsules or tablets, or oral liquid preparations. If desired, tablets may be coated by standard aqueous or non-aqueous techniques.

For example, a tablet may be prepared by compression or molding, optionally, with one or more accessory ingredients. Compressed tablets may be prepared by compressing in a suitable machine the active ingredient in a free-flowing form such as powder or granules, optionally mixed with a binder, lubricant, inert diluent, granulating agent, surface active agent, dispersing agent, or the like. Molded tablets may be made by molding, in a suitable machine, a mixture of the powdered compound moistened with an inert liquid diluent. In one embodiment, each tablet, capsule, cachet, or gel cap contains from about 0.5 mg to about 500 mg of the active ingredient, while in another embodiment, each tablet contains from about 1 mg to about 250 mg, preferably about 10 to 50 mg of the active ingredient; see also supra. The amount of active ingredient found in the composition, however, may vary depending on the amount of active ingredient to be administered to the patient.

The metal oxide, or a suitable derivative thereof, may be formulated as a pharmaceutical composition in a soft elastic or hard gelatin capsule unit dosage form by using conventional methods well known in the art, such as in Ebert, Pharm. Tech. 1 (1977), 44-50; see also the Example. Soft elastic gelatin capsules have a soft, globular gelatin shell somewhat thicker than that of hard gelatin capsules, wherein a gelatin is plasticized by the addition of plasticizing agent, e.g., glycerin, sorbitol, or a similar polyol. The hardness of the capsule shell may be changed by varying the type of gelatin used and the amounts of plasticizer and water. The soft gelatin shells may contain a preservative, such as methyl-and propylparabens and sorbic acid, to prevent the growth of fungi. The active ingredient may be dissolved or suspended in a liquid vehicle or carrier, such as vegetable or mineral oils, triglycerides, surfactants such as polysorbates, or a combination thereof.

The metal oxide can also be delivered via a controlled release delivery vehicle. In a preferred embodiment, the controlled release vehicle includes a polymeric material, delivered or surgically implanted at or near the lesion site. One of ordinary skill in the art will be familiar with controlled release means and delivery devices, such as those described in US Patent Nos.: 3,845,770; 3,916,899; 3,536,809; 3,598,123; 4,008,719; 5,674,533; 5,059,595; 5,591,767; 5,120,548; 5,073,543; 5,639,476; 5,354,556; and 5,733,566, the disclosures of which are hereby incorporated herein by express reference thereto. These pharmaceutical compositions can be used to provide slow or controlled-release of the active ingredient therein using, for example, hydropropylmethyl cellulose in varying proportions to provide the desired release profile, other polymer matrices, gels, permeable membranes, osmotic systems, multilayer coatings, microparticles, liposomes, microspheres, or the like, or a combination thereof. Suitable controlled-release formulations available to those of ordinary skill in the art, including those described herein, may be readily selected for use with the the metal oxide compositions of the invention. Thus, single unit dosage forms suitable for topical, parenteral, or oral administration, such as infusions, intravenous drips, gels, lotions, cremes, tablets, capsules, gelcaps, caplets, and the like, that are adapted for controlled release are encompassed by the present invention.

All controlled-release pharmaceutical products have a common goal of improving drug therapy over that achieved by their non-controlled counterparts. Ideally, the use of an optimally designed controlled-release preparation in medical treatment is characterized by a minimum of drug substance being employed to cure or control the condition in a minimum amount of time. Advantages of controlled-release formulations may include: 1) extended activity of the drug; 2) reduced dosage frequency; and 3) increased patient compliance.

Most controlled-release formulations are designed to initially release an amount of drug that promptly produces the desired therapeutic effect, and gradual and continual release of other amounts of drug to maintain this level of therapeutic effect over an extended period of time. In order to maintain this constant level of drug in the body, the drug should be released from the dosage form at a rate that will replace the amount of drug being metabolized and excreted from the body.

The controlled release of the active ingredient may be stimulated by various induces, for example pH, temperature, enzymes, water, or other physiological conditions or compounds.

The pharmaceutical compositions for use in the present invention include the metal oxide or a derivative thereof, as the active ingredient, and may also contain a pharmaceutically acceptable carrier, and optionally, other therapeutic ingredients and/or preservatives such as surfactants. Suitable derivatives include any available "pharmaceutically acceptable salts", which refer to a salt prepared from pharmaceutically acceptable non-toxic acids including inorganic acids, organic acids, solvates, hydrates, or clathrates thereof. Examples of such inorganic acids are hydrochloric, hydrobromic, hydroiodic, nitric, sulfuric, and phosphoric. Appropriate organic acids may be selected, for example, from aliphatic, aromatic, carboxylic and sulfonic classes of organic acids, examples of which are formic, acetic, propion, succinic, citric, fumaric, gluconic, isethionic, lactic, malic, mucic, tartaric, para-toluenesulfonic, glycolic, glucuronic, maleic, furoic, glutamic, salicylic, mandelic, methanesulfonic, ethanesulfonic, benzenesulfonic (besylate), sulfanilic, alginic, galacturonic, and the like. Particularly preferred acids are phosphoric, methanesulfonic, and glycolic.

In a preferred embodiment, the pharmaceutical composition of the present invention is intended for the treatment of HIV/AIDS and may further comprise a therapeutically effective agent. As demonstrated in the example, the pharmaceutical compositions of the present invention are particularly useful for the treatment of HIV and the treatment of consequent pathological conditions such as AIDS. Preventing AIDS, treating AIDS, delaying the onset of AIDS, or preventing or treating infection by HIV is defined as including, but not limited to, treatment of a wide range of states of HIV infection including both symptomatic and asymptomatic, and actual or potential exposure to HIV. For example, the compositions of this invention are useful in treating infection by HIV after suspected past exposure to HIV by such means as blood transfusion, exchange of body fluids, bites, accidental needle stick, or exposure to patient blood during surgery.

The present invention includes a method for inhibiting HTV in a subject in need thereof which comprises administering to the subject the pharmaceutical composition of the present invention as originally defined above. The invention also includes a method for preventing or treating HIV infection or for preventing, treating or delaying the onset of AIDS in a subject in need of such treatment, which comprises administering to the subject the pharmaceutical composition of the present invention as originally defined above. In these methods, the compositions of the present invention can optionally be employed in combination with one or more HIV/AIDS treatment agents selected from HIV/AIDS antiviral agents, anti-infective agents, and immunomodulators. Embodiments of these methods include the methods as just described wherein the pharmaceutical composition is a pharmaceutical composition as set forth in any one of the foregoing embodiments or in an aspect thereof.

As mentioned before, the pharmaceutical compositions of this invention are preferably administered orally in a suitable dosage form, such as capsules or compressed tablets. The compositions can be administered in a dosage range of from about 0.01 to about 10 mg/kg of mammal (e.g., human) body weight per day in a single dose or in divided doses. One preferred dosage range is from about 0.1 to about 5 mg/kg body weight per day orally in a single dose or in divided doses. Another preferred dosage range is from about 0.2 to about 2.5 mg/kg body weight orally in single or divided doses. For oral administration, the compositions can suitably be provided in the form of tablets or capsules containing from about 0.1 to about 1000 milligrams of the active ingredient, particularly 1, 5, 10, 15, 20, 25, 50, 75, 100, 150, 200, 250, 300, 400, 500, 600, and 800 milligrams of the active ingredient for the symptomatic adjustment of the dosage to the patient to be treated. A preferred oral dose is an encapsulated or tabletted composition of the invention containing from about 5 to about 100 mg of active ingredient. In another preferred dose, the encapsulated or tabletted composition contains from about 10 to about 50 mg of active ingredient; i.e. metal oxide.

The specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy. As noted earlier, although the active drug substance (i.e. active ingredient) can be administered as a salt, all references herein to the amount of active ingredient are to the free acid (alternatively referred to herein as the "free phenol") or free base form of the compound, unless expressly stated to the contrary in a particular context.

As noted above, the present invention is also directed to use of the pharmaceutical compositions of the present invention with one or more agents useful in the treatment of HIV infection or AIDS. For example, the compositions of this invention may be effectively administered, whether at periods of pre-exposure and/or post-exposure, in combination with effective amounts of one or more of the HIV/AIDS antivirals, imunomodulators, antiinfectives, or vaccines useful for treating HIV infection or AIDS. Suitable agents include the HIV antivirals as cited in Table 1 of international application WO01/38332 and WO03/086319, respectively, which also disclose other antivirals plus immunomodulators, anti-infectives, or vaccines useful for treating HIV infection or treating or delaying the onset of AIDS that can be used in combination with the pharmaceutical compositions of the present invention, which are herein incorporated by reference in its entirety. It will be understood that the scope of combinations of compositions of this invention with HIV/AIDS antivirals, immunomodulators, anti-infectives or vaccines is not limited to those listed in the mentioned Tables, but includes in principle any combination with any other pharmaceutical composition useful for the treatment of AIDS. The HIV/AIDS antivirals and other agents will typically be employed in these combinations in their conventional dosage ranges and regimens as reported in the art, including the dosages described in the Physicians' Desk Reference, 54th edition, Medical Economics Company, 2000. The dosage ranges of the active drug substances in the pharmaceutical compositions of the present invention employed in these combinations are suitably the same as those set forth above.

When the composition of the invention is provided in combination with one or more other active agents (e.g., antiviral agents useful for treating HIV infection or AIDS), "administration" and its variants are each understood to include concurrent and sequential provision of the composition and other agents. Thus, for example, administration of the pharmaceutical composition of the invention in combination with another antiviral agent means that the composition of the invention can be administered before, at the same time, or after the other agent.

As is evident from the example and the description above, the present invention also relates to the use of at least one metal oxide as defined hereinbefore for the preparation of a pharmaceutical composition for the treatment of various diseases including a tumor, an autoimmune disease, allergy, a degenerative disease, vascular disease, metabolic disease, an infectious disease, or for wound healing.

Hence, the compositions and methods of the invention advantageously prevent, treat, or manage infectious diseases or diseases which result from alteration of cellular events, in particular disease that involve the immune system in any kind. The conditions against which the compounds, such as metal oxides, of the present invention have utility include, but are not limited to, Madura foot, actinomycosis, oral actinomycosis, anthrax, food poisoning, botulism, wound infections, pseudomembranous colitis, colitis, gas gangrene, gangrene, tetanus, diphtheria, pharyngeal diphtheria, pleomorphic laryngeal diphtheria, cutaneous diphtheria, endocarditis, bacteremia, urinary tract infections, listerosis, meningitis, miscarriage, narcodiosis, acne, skin lesions, abscesses, toxic shock syndrome, prosthesis contamination, dental caries, plaque, gum disease, gingivitis, subacute endocarditis, bacterial pneumonia, otitis, sinusitis, cat scratch fever, septicemia, abdominal and pelvic abscesses, Oroya fever, systemic Oroya fever, verruga peruana, cutaneous verruga peruana, whooping cough, Lyme disease, epidemic relapsing fever, brucellosis, granuloma inguinale, granulomatic donovanosis, gastroenteritis, nosocomial infections, tularemia, bacterial vaginitis, urethritis, bacterial conjunctivitis, chancroid, otitis media, chronic gastritis, peptic ulcer, diarrhea, Legionnaires' disease, leptospirosis, gonorrhea, arthritis, periodontal disease, salmonellosis, typhoid fever, shigellosis, rat bite fever, pharyngitis, scarlet fever, syphilis, cholera, Asiatic cholera, Yersina arthritis, bubonic plague, chronic pulmonary disease, Hansen's disease, leprosy, tuberculosis, dermal tuberculosis, psittachosis, ornithosis, conjunctivitis, trachoma, lymphogranuloma venereum, genital tract infections, Q fever, primary atypical pneumonia, rickettsial pox, typhus, epidemic typhus, Rocky Mountain spotted fever, tsutsugamushi fever, nongonococcal urethritis, human erlichiosis, meningococcal meningitis, skin infections, corneal infections, external ear infections, candidiasis, monoiliasis, thrush, candidosis, mucositis, bacteremia, hepatitis, hepatitis A, hepatitis B, hepatitis C, hepatitis E, coccidiomycosis, lymphadenitis, balantidiasis cryptosporidosis, amoebiasis, amoebic dysentery, giardiasis, giardia enteritis, leishmaniasis, Kala-azar, malaria, toxoplasmosis, trypanosomiasis, Chagas disease, African sleeping sickness, dengue, Japanese encephalitis, Rift Valley fever, Ebola hemorrhagic fever, Venezuelan hemorrhagic fever, hantavirus pulmonary syndrome, hemorrhagic fever with renal syndrome, cytomegalovirus infection, poliomyelitis, West Nile virus disease, influenza, measles, condyloma, encephalitis, ankylosing spondylitis, arteritis, inflammatory bowel disease, polyarteritis nodosa, rheumatic fever, systemic Lupus erythematosus, Alzheimer' s disease, multiple sclerosis, osteoporosis, Crohn' s disease, strep throat, yellow fever, eczema, psoriasis, dermatitis, disease-induced skin ulcers, undefined tropical diseases, shingles, rashes, heat rashes, bedsores, cold sores, blisters, boils, herpes simplex, acne, pimples, skin chafing, skin cracking, itchiness, skin peeling, warts, one or more symptoms thereof, or any combination thereof.
In another embodiment, the condition includes HIV (AIDS), or one or more symptoms. It should be understood that the invention includes the use of the compounds or compositions to prevent, treat, or manage each of these conditions individually or multiple conditions concurrently or sequentially. Thus, the prevention, treatment, or management of each condition should be understood as a separate embodiment.
The pathogens which may be killed by, or the growth or proliferation of which may be halted, diminished, or inhibited by the metal oxides of the present invention include, but are not limited to, gram-positive bacilli and cocci; gram-negative bacilli and cocci; acid-fast bacteria; other bacteria; fungi; parasitic microbes, e.g., protozoa; and viruses.
Examples of gram-positive bacilli and cocci include, but are not limited to, Actinomedurae, Actinomyces israelii, Bacillus anthracis, Bacillus cereus, Clostridium botulinum, Clostridium difficile, Clostridium perfringens, Clostridium tetani, Corynebacterium, Enterococcus faecalis, Listeria monocytogenes, Nocardia, Propionibacterium acnes, Staphylococcus aureus, Staphylococcus epidermalis, Streptococcus mutans, Streptococcus pneumoniae, and combinations thereof.
Examples of gram-negative bacilli and cocci include, but are not limited to, Afipia felis, Bacteriodes, Bartonella bacilliformis, Bortadella pertussis, Borrelia burgdorferi, Borrelia recurrentis, Brucella, Calymmatobacterium granulomatis, Campylobacter, Escherichia coli, Francisella tularensis, Gardnerella vaginalis, Haemophilius aegyptius. Haemophilius ducreyi, Haemophilius inifluenziae, Heliobacter pylori, Legionella pneumophila, Leptospira interrogans, Neisseria meningitidia, Porphyromonas gingivalis, Providencia sturti, Pseudomonas aeruginosa, Salmonella enteridis, Salmonella typhi, Serratia marcescens, Shigella boydii, Streptobacillus moniliformis, Streptococcus pyogenes, Treponema pallidum, Vibrio cholerae, Yersinia enterocolitica, Yersinia pestis, and combinations thereof.
Examples of acid-fast bacteria include, but are not limited to, Myobacterium avium, Myobacterium leprae, Myobacterium tuberculosis, and combinations thereof.
Examples of other bacteria not falling into the other three categories include, but are not limited to, Bartonella henseiae, Chlamydia psittaci, Chlamydia trachomatis, Coxiella bumetii, Mycoplasma pneumoniae, Rickettsia akari, Rickettsia prowazekii, Rickettsia rickettsii, Rickettsia tsutsugamushi, Rickettsia typhi, Ureaplasma urealyticum, Diplococcus pneumoniae, Ehrlichia chafensis, Enterococcus faecium, Meningococci, and combinations thereof.
Examples of fungi include, but are not limited to, Aspergilli, Candidae, Candida albicans, Coccidioides immitis, Cryptococci, and combinations thereof.
Examples of parasitic microbes include, but are not limited to, Balantidium coli, Cryptosporidium parvum, Cyclospora cayatanensis, Encephalitozoa, Entamoeba histolytica, Enterocytozoon bieneusi, Giardia lamblia, Leishmaniae, Plasmodii, Toxoplasma gondii, Trypanosomae, trapezoidal amoeba, and combinations thereof.
Examples of viruses include, but are not limited to, Arboviruses, Ebola virus, Guanarito virus, Hanta virus, Hantaan virus, Hepatitis A, Hepatitis B, Hepatitis C, Hepatitis E, other Hepatitis viruses, Herpes-type viruses, Poliovirus, CMV, West Nile virus, Echo virus, and combinations thereof.
The anti-pathogenic or non-pathogenic compositions o the present invention may optionally further include the use of one or more additional therapeutic agents known to treat a condition, or a symptom thereof. Examples of such additional therapeutic agents include, but are not limited to, chelating agents, vitamins, minerals, silica hydride microclusters, analgesics, Sambucol™, aspirin, and the like.

The administration of one or more active ingredients and/or optional therapeutic agent(s) in accordance with the methods of the invention may occur together, concurrently but separately, sequentially, or a combination thereof. The optional additional therapeutic agent is generally a compound other than a metal oxide compound.

The appropriate concentration of the therapeutic agent might be dependent on the particular agent. The therapeutically effective dose has to be compared with the toxic concentrations; the clearance rate as well as the metabolic products play a role as do the solubility and the formulation. Therapeutic efficacy and toxicity of compounds can be determined by standard pharmaceutical procedures in cell cultures or experimental animals, e.g., ED50 (the dose therapeutically effective in 50 % of the population) and LD50 (the dose lethal to 50 % of the population). The dose ratio between therapeutic and toxic effects is the therapeutic index, and it can be expressed as the ratio, LD50/ED50.

These and other embodiments are disclosed and encompassed by the description and example of the present invention. Further literature concerning any one of the materials, methods, uses and compounds to be employed in accordance with the present invention may be retrieved from public libraries and databases, using for example electronic devices. For example the public database "Medline" may be utilized, which is hosted by the National Center for Biotechnology Information and/or the National Library of Medicine at the National Institutes of Health. Further databases and web addresses, such as those of the European Bioinformatics Institute (EBI), which is part of the European Molecular Biology Laboratory (EMBL) are known to the person skilled in the art and can also be obtained using internet search engines. An overview of patent information in biotechnology and a survey of relevant sources of patent information useful for retrospective searching and for current awareness is given in Berks, TIBTECH 12 (1994), 352-364.

The above disclosure generally describes the present invention. Several documents are cited throughout the text of this specification. The contents of all cited references (including literature references, issued patents, published patent applications as cited throughout this application and manufacturer's specifications, instructions, etc) are hereby expressly incorporated by reference; however, there is no admission that any document cited is indeed prior art as to the present invention.

A more complete understanding can be obtained by reference to the following specific example which is provided herein for purposes of illustration only and are not intended to limit the scope of the invention.

### EXAMPLE

Detailed descriptions of conventional methods, such as those employed herein can be found in the cited literature; see also Remington: The Science and Practice of Pharmacy (20th Edition, 2000) by Gennaro et al. (eds.) ISBN 0-683-306472 and "The Merck Manual of Diagnosis and Therapy" Seventeenth Ed. ed by Beers and Berkow (Merck & Co., Inc. 2003).

### Example: Clinical trial with patients suffering from HIV/AIDS

5 persons, 2 women (1 of them nine months pregnant) and 3 men, participated in the trial. Their condition was to be described as terminal stage of AIDS with the following symptoms:
- all 5 patients suffered from Pneumocystis carinii
- 4 patients suffered from hepatitis C
- 3 patients suffered from Kaposi's sarcoma
- 3 patients suffered from CMV
- both women suffered from candidiasis
- all patients suffered from a very severe cough
- all patients suffered from severe emaciation
For verification of the results, different tests have been conducted (ELISA, PCR, p24-antigen-test). The high-level burden of virus (PCR) confirmed the symptomatology. All patients were HIV-1 and HIV-2 (ELISA, Western Blot, sub-typing) positive. It may hereby be of interest to mention that HIV-2 is the more uncommon type, mainly occurring in Western Africa, that has proved to be more resistant to several of the common therapies like e.g. reverse-transcriptase-inhibitors.

Only red iron (III), i.e. ferric oxide (Fe₂O₃) of 99.7 % purity was used. It was administered in stomach-soluble hard gelatin capsules in doses of 400 mg and 20 mg. The agent can be filled into the preferred stomach-soluble hard gelatin capsules by a chemist or a manufacturer in the above mentioned dosage without adding aiding substances. For general methods of preparing such capsules and other means, e.g. tablets see for example the standard pharmacy book Remington: The Science and Practice of Pharmacy (20^{th} Edition, 2000) by Gennaro et al. (eds.) ISBN 0-683-306472, in particular Chapter 45, p. 885ff. An extensive product range - encompassing more than 250 gelatin types and gelatin-/collagen hydrolysates - satisfying the requirements of therapeutically effective gelatin capsules and available as tailor-made solutions are provided by, for example GELATINE PRODUCTS Ltd., Knutsford, Great Britain. Therapy started with the administration of 400 mg once a day on an empty stomach for 5 days.

The following symptoms were noted:
- intense red coloring of the stool of all patients on all 5 days
- all 5 patients complained throughout about a headache of medium intensity
- 3 of the 5 patients complained about gastro-intestinal conditions of medium intensity
- one of the patients got diarrhea
- one of the female patients complained about light nausea

From day 6 on the dose was reduced to 100 mg (5 times a day 20 mg) per day, also on an empty stomach. About every 2 hours, the patients received a tablet. This form of administration was tolerated considerably better by all the patients. A distinct reduction of red coloring of the stool could be observed. None of the patients complained about side effects anymore. 4 of 5 patients reported of a systemic increase in blood circulation. After 4 weeks of therapy, the convalescence of all patients was already considerably advanced and after about 6 weeks, all patients were free of symptoms.
After 8 weeks of therapy the same tests as mentioned above were conducted once again and the following results have been obtained:
- PCR: no virus detectable anymore
- p24: no antigens detectable anymore
- ELISA & Western Blot: antibodies reduced down to a level of invisibility.

The blood count results were:
- no hepatitis C detectable anymore
- no CMV detectable anymore

A pap smear also showed that the amount of Candida albicans in both women was reduced down to a normal degree again. Furthermore, during therapy the child was born and remained virus-free.
Even 3 months after this 8-week-therapy the laboratory results remained unaltered. The patients were healthy and could pursue their former occupations again.

As the intestinal absorbability and the therapeutic effectiveness of this compound, which health authorities and scientific experts up to now consider non-absorbable and therapeutically less valuable, could unmistakably be proved by the present results, the officially valid recommendations for the supply with iron compounds, which settle down at around 8 and 18 mg per day, should above all be obeyed when administering and dosing. The maximum absorption capacity of the body lies around 1 to 3 mg per day in a healthy person.

The side effects occurring with 400 mg per day, like headache and nausea, are also the officially known side effects of iron overdosing reported in the literature. 400 mg "administered without any reservations" as claimed and recommended in international application WO95/35113 could be of fatal consequences in an infant or a small child.

The observation that the single administration of a very high dose led to an increased red coloring of the stool in all patients compared to graduated administration of smaller amounts during the whole day correlates with personal observations that 400 mg once a day will color the stool more intensely red than, e.g., 50 mg eight times a day. In accordance with the present invention it is presumed that the body has momentary maximum values of absorption, which are not identical with the daily maximum values of absorption, so that a graduated administration during the whole day, apart from higher tolerability, can simulate the physiologic situation in a better way and therefore leads to better absorption.
As it has shown that already with considerably lower dosage (here 100 mg Fe₂O₃ a day in 20 mg dosage units; the required minimum dosage is probably even lower) an optimal result without side effects could be achieved, the recommendation of a single 400 mg dosage unit per day does not seem to be in error, probably because of the fact that this dose recommendation in international application WO95/35113 is based on in vitro experiments, which do not reflect the complexity of the animal, in particular human body.

Should a higher dosage be required in several extreme cases, the optimal lowest possible dosage should slowly be approached by successive administration of a low dosage (or, if possible, by means of a form of slow-release-tablet with delayed release of the agent) in order to achieve the optimal therapeutic success and to avoid unnecessary overload of the gastro-intestinal system as well as side effects, which naturally is more than desirable in an diseased and weakened organism. For the preparation of slow-release compositions see also the mentioned textbook Remington, supra. External application such as topical application in form of ointment or cream surely is also possible.

In view of the fact that besides HIV and the other viruses also the opportunistic infections could be successfully treated and at least be ameliorated the actual range of metal oxide therapy envisaged by the present invention of course is wider than the treatment of HIV/AIDS only, and comprises, for example:
- viral diseases like HIV, hepatitis, CMV, herpes, influenza, etc.
- bacterial infections like pneumonia, tuberculosis,
- cancerous diseases
- forms of mycosis
- autoimmune diseases like arthritis, lupus, etc.
- degenerative diseases of the musculoskeletal system like degenerative arthritis
- blood circulation disorders
- metabolic derailments like diabetes, etc.
- allergic diseases
- parasitic diseases like malaria, etc.
- enhanced wound healing

With all these diseases, the metal oxide composition such as preferably iron(III) oxide is sufficient either in form of a single preparation or for adjuvant therapy in combination with other drugs. While application in human is preferred, veterinary medicine surely is very useful as well.

## Claims

1. A pharmaceutical composition comprising in a dosage unit as the effective ingredient a metal oxide, and optionally a pharmaceutically acceptable carrier.

2. The pharmaceutical composition of claim 1, wherein said metal is iron.

3. The pharmaceutical composition of claim 1 or 2, wherein said metal oxide is iron oxide Fe₂O₃ or hydrated iron oxide Fe₂O₃ (H₂O).

4. The pharmaceutical composition of any one of claims 1 to 3, wherein said dosage unit comprises about 1 to 350 mg of the metal oxide, preferably about 10 to 50 mg.

5. The pharmaceutical composition of any one of claims 1 to 4, wherein said metal oxide is present in an amount sufficient to administering to the subject from about 0.1 to about 5 mg/kg/day of said metal oxide, preferably wherein said pharmaceutical composition is designed for administration twice to eight times a day.

6. The pharmaceutical composition of any one of claims 1 to 5, wherein said metal oxide is present in said dosage unit from about 0.15 to about 0.5 mg/kg.

7. The pharmaceutical composition of any one of claims 1 to 6, comprising a further therapeutically effective agent and/or a preservative.

8. Use of a metal oxide in a dosage unit as defined in any one of claims 1 to 6 for the preparation of a pharmaceutical composition for the treatment of a tumor, an autoimmune disease, an allergy, a degenerative disease, a vascular disease, a metabolic disease, an infectious disease except AIDS, or for wound healing.

9. The use of claim 8, wherein said infectious disease results from the infection by a virus or microorganism.

10. The use of claim 9, wherein said virus is HCV or CMV.
